# EUROPEAN PATENT APPLICATION

(11) **EP 4 527 284 A1**
(43) Date of publication of application: **26.03.2025**
(21) Application number: 24182075.2
(22) Date of filing: 13.06.2024
(51) Int. Cl.: A61B 1/00, A61B 50/10, A61B 50/20, A61B 90/98

(54) **FLEXIBLE ENDOSCOPE STORAGE HOOK, CABINET AND SYSTEM**

(30) Priority: 22.09.2023 US 202363539842 P
(71) Applicant: Olympus Winter & Ibe GmbH, 22045 Hamburg (DE)
(72) Inventor: SCHROEDER, Stefan, 20459 Hamburg (DE)
(74) Representative: Seemann & Partner Patentanwälte mbB

(57) **Abstract**

The present disclosure relates to a flexible endoscope storage hook, cabinet and system. The flexible endoscope storage hook comprises a receptacle for a flexible endoscope, an endoscope presence sensor designed for detecting a placement or removal of a flexible endoscope in the receptacle, and a controller.

## Description

The present disclosure relates to a flexible endoscope storage hook.

The reprocessing of flexible endoscopes requires an adequate drying and storage after high-level disinfection. Customers can choose from a variety of product offerings such as drying and/or storage cabinets. Some customers refrain from buying such fully equipped drying and/or storage cabinets and instead construct their own storage cabinets e.g. from standard furniture or within a dedicated room. These self-built solutions can be compliant with latest standards and are cost-effective.

However, these self-built solutions lack several features compared to drying and storage cabinets. Especially, the individual track and trace of a single endoscope is not possible or only with additional manual steps. Control parameters such as storage time, operator identification or documentation of environmental parameters such as temperature and humidity, which are customary in commercially available drying and/or storage cabinets, cannot be processed automatically. Therefore, operating a reprocessing workflow with self-built storage solutions entails substantial disadvantages in regards to documentation and process control. This will result in negative impact on overall infection prevention and control.

It is an object to provide a means for avoiding the above-described problems even in self-built storage solutions.

Such object can be solved by a flexible endoscope storage hook comprising a receptacle for a flexible endoscope, an endoscope presence sensor designed for detecting a placement or removal of a flexible endoscope in the receptacle, and a controller.

In embodiments, the controller is designed and equipped for process control and/or documentation. Such process control and/or documentation may comprise one or more of track and trace of one or more individual flexible endoscopes, control parameters such as storage time, operator identification or documentation of environmental parameters such as temperature and humidity.

Such a hook having its own sensor and controller can also be installed in commercially available, simple storage cabinets and take over the aforementioned tasks for process control and/or documentation.

The storage hook is equipped with means for storage and process control that is commonly implemented into commercially available drying and storage cabinets. In order to implement this functionality, in embodiments the controller comprises a CPU, a display and a wireless connection unit. Wireless connectivity such as Wi-Fi or Bluetooth may be used to integrate one or more flexible endoscope storage hooks into a system providing centralized endoscope storage control.

In various embodiments, the controller may comprise one or more of a temperature sensor, a humidity sensor, an internal clock, and an RFID readout configured for reading out RFID equipped endoscopes and/or users, which all are useful for documentation and process control.

In further embodiments, the controller may comprise a self-contained power supply, in particular a battery, or a connector to an external power supply.

The endoscope presence sensor may in embodiments be one of an optical sensor, a contact sensor, in particular a switch, a weight sensor and a deformation sensor being located on or in vicinity to the receptacle. An optical sensor may be a photoelectric barrier to be blocked by a flexible endoscope being held by the receptacle or a photodiode or other photosensor that may be covered by an endoscope when held by the receptacle. A contact sensor may react to being in physical contact with an endoscope in the receptacle. While a weight sensor may be located to measure the weight of an endoscope held by the receptacle, a deformation sensor may be integrated into the receptacle to measure the deformation of the receptacle under the weight of an endoscope held by the receptacle. The types of sensors to be used for detecting the presence or absence of an endoscope is not limited to this list of sensors.

In embodiments, the flexible endoscope storage hook comprises fixing means, in particular prefabricated screw holes or a clamp, designed for aiding a fixing off the hook to a counterpart in or at a storage device or room.

The object can also be solved by a flexible endoscope storage cabinet having one or more flexible endoscope storage hooks according to the previous disclosure, and by a flexible endoscope storage system having a central computer and one or more flexible endoscope storage hooks according to the previous disclosure, the central computer being designed and configured to connect or be connected to the one or more flexible endoscope storage hooks, to receive data from the one or more flexible endoscope storage hooks and to centrally process the received data for documentation of storage of one or more flexible endoscopes.

Further features will become evident from the description of embodiments, together with the claims and the appended drawings. Embodiments can fulfill individual features or a combination of several features.

The embodiments described below, without restricting the general intent of the invention, based on exemplary embodiments, wherein reference is made expressly to the drawings with regard to the disclosure of all details that are not explained in greater detail in the text.

In the drawings:
Fig. 1 illustrates schematically an embodiment of a flexible endoscope storage hook,
Fig. 2 illustrates schematically an embodiment of a controller of a flexible endoscope storage hook,
Fig. 3 illustrates schematically an embodiment of a flexible endoscope storage cabinet and
Fig. 4 illustrates schematically an embodiment of a flexible endoscope storage system.

In the drawings, the same or similar types of elements or respectively corresponding parts are provided with the same reference numbers in order to prevent the item from needing to be reintroduced.

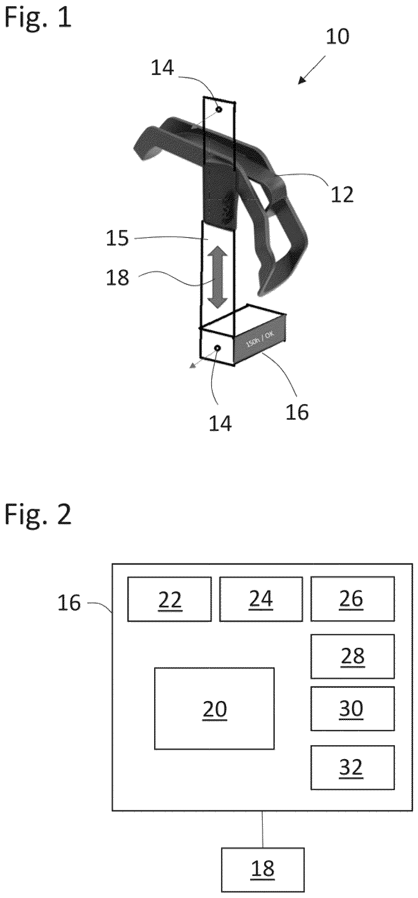

Fig. 1 illustrates schematically an embodiment of a flexible endoscope storage hook 10 having a receptacle 12 designed to accommodate a flexible endoscope (not shown) for the purpose of storing the endoscope until further use. The storage hook 10 has a back plate 15 with screw holes 14 for affixing the storage hook 10 a wall of a cabinet or a storage room.

A sensor 18 is designed and configured to sense whether a flexible endoscope is present in or at receptacle 12 or not.

The storage hook 10 also comprises a controller 16 receiving the signal from sensor 18 and having components rendering the controller 16 capable of performing several functions that are built-in to commercially available drying and storage cabinets such as track and trace of individual endoscopes, controlling parameters such as storage time, operator identification or documentation of environmental parameters such as temperature and humidity. This, in essence, allows the user to perform operating a reprocessing workflow as it would be possible with commercially available drying and storage cabinets, thereby alleviating the substantial disadvantages self-built storage cabinets have with regard to documentation and process control and the negative impact this has on overall infection prevention and control.

Figure 2 illustrates components of controller 16 that enable controller 16, and by extension storage hook 10, to carry out several of the above-described functions. A central unit of controller 16 is a CPU 20 that is connected to a number of peripheral units, several or all of which may be present in controller 16. Controller 16 may have a display 22 configured to show information about an endoscope, storage related or other information to a user, either permanently or on request. Such a display 22 may be a touchscreen device, obviating the need for a separate manual input unit, which may alternatively be present in case the display 22 does not function as a touchscreen device.

Controller 16 may also comprise a wireless connection unit 24, for example a Wi-Fi or Bluetooth unit configured to connect controller 16 into a wireless local area network to which a central control unit, for example a computer, may be connected that performs a coordinating function across several similar flexible endoscope storage hooks 10.

Controller 16 may also be equipped with one or more of a temperature and/or timidity sensor 26, which may be a temperature sensor and a humidity sensor, internal clock 28, an RFID readout unit 30 and a power supply 32, which may be a self-contained power supply such as a battery or a connection to an external power supply. The RFID readout unit 30 may be configured to readout RFID tags of endoscopes for identification of individual endoscopes and/or RFID tags worn by users to document which user has used the storage hook 10 at what times and possibly with which endoscope.

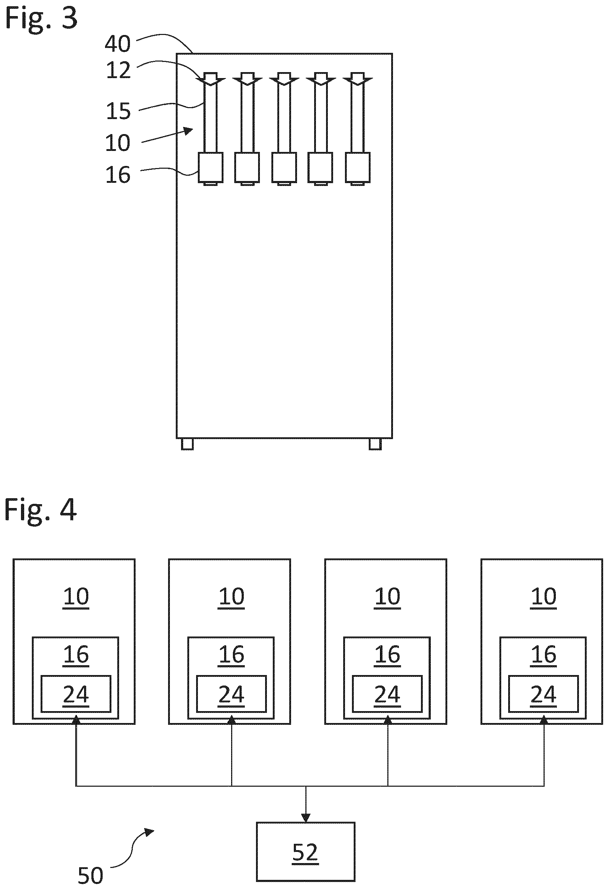

Fig. 3 illustrates schematically an embodiment of a flexible endoscope storage cabinet 40. The flexible endoscope storage cabinet 40 is in essence a cabinet with a multiple of flexible endoscope storage hooks 10 that may be configured according to Fig. 1 and Fig. 2 affixed to its back wall. The cabinet 40 itself has no controller of its own. Instead process control and documentation are provided by the flexible endoscope storage hooks 10 that are equipped with their own controllers 16, which have built-in process control and documentation functionality.

Fig. 4 illustrates schematically an embodiment of a flexible endoscope storage system 50 having a central computer 52 and several flexible endoscope storage hooks 10 according to the present disclosure, for example such as are shown in Figs. 1 and 2 and used in Fig. 3. Data connectivity between the hooks 10 and the central computer 52 may be provided by wireless connectivity units 24 contained in the respective controllers 16 of the respective flexible endoscope storage hooks 10 of system 50. The central computer 52 may be configured to provide a centralized documentation and process control needed in the context of clinical endoscopy with multiple endoscopes.

Alternatively, a flexible endoscope storage system may be configured without a central computer 52. In that case, the several flexible endoscope storage hooks 10 may interconnect with each other via their wireless connection units 24 and provide either a distributed process control and documentation or, in the context of establishing a connection, broker a specific one of the flexible endoscope storage hooks 10 to act as a central process control and documentation unit receiving and processing data from all the other connected flexible endoscope storage hooks 10, thus providing the same functionality as the central computer 52 of the embodiment of Fig. 4.

While there has been shown and described what is considered to be embodiments of the invention, it will, of course, be understood that various modifications and changes in form or detail could readily be made without departing from the spirit of the invention. It is therefore intended that the invention be not limited to the exact forms described and illustrated, but should be constructed to cover all modifications that may fall within the scope of the appended claims.

### List of Reference Signs

- 10: flexible endoscope storage hook
- 12: receptacle
- 14: screw hole
- 15: back plate
- 16: controller
- 18: sensor
- 20: CPU
- 22: display
- 24: wireless connection unit
- 26: temperature sensor
- 28: internal clock
- 30: RFID readout unit
- 32: power supply
- 40: storage cabinet
- 50: storage system
- 52: computer

## Claims

1. Flexible endoscope storage hook comprising a receptacle for a flexible endoscope, an endoscope presence sensor designed for detecting a placement or removal of a flexible endoscope in the receptacle, and a controller.

2. The flexible endoscope storage hook of claim 1, wherein the controller is designed and configured for process control and/or documentation.

3. The flexible endoscope storage hook of claim 1, wherein the controller comprises a CPU, a display and a wireless connection unit.

4. The flexible endoscope storage hook of claim 1, wherein the controller comprises at least one of a temperature sensor and a humidity sensor.

5. The flexible endoscope storage hook of claim 1, wherein the controller is equipped with an internal clock.

6. The flexible endoscope storage hook of claim 1, wherein the controller comprises an RFID readout configured for RFID equipped endoscopes and/or users.

7. The flexible endoscope storage hook of claim 1, wherein the controller comprises a self-contained power supply, in particular a battery, or a connector to an external power supply.

8. The flexible endoscope storage hook of claim 1, wherein the endoscope presence sensor is one of an optical sensor, a contact sensor, in particular a switch, a weight sensor and a deformation sensor being located on or in vicinity to the receptacle.

9. The flexible endoscope storage hook of claim 1, further comprising fixing means, in particular prefabricated screw holes or a clamp, designed for aiding a fixing off the hook to a counterpart in or at a storage device or room.

10. Flexible endoscope storage cabinet having one or more flexible endoscope storage hooks according to claim 1.

11. Flexible endoscope storage system having a central computer and one or more flexible endoscope storage hooks according to claim 1, the central computer being designed and configured to connect or be connected to the one or more flexible endoscope storage hooks, to receive data from the one or more flexible endoscope storage hooks and to centrally process the received data for documentation of storage of one or more flexible endoscopes.
